# EUROPEAN PATENT SPECIFICATION

(11) **EP 3 431 954 B1**
(45) Date of publication and mention of the grant of the patent: **05.08.2026**
(21) Application number: 18174939.1
(22) Date of filing: 29.05.2018
(51) Int. Cl.: G01N 1/22, G01N 33/00

(54) **ACTUATING AND SENSING MODULE**
BETÄTIGUNGS- UND MESSMODUL
MODULE D'ACTIONNEMENT ET DE DÉTECTION

(30) Priority: 10.07.2017 TW 106123108
(43) Date of publication of application: 23.01.2019
(73) Proprietor: Microjet Technology Co., Ltd, Hsinchu (TW)
(72) Inventor: MOU, Hao-Jan, Hsinchu (TW); HSUEH, Ta-Wei, Hsinchu (TW); CHEN, Shih-Chang, Hsinchu (TW); MO, Li-Pang, Hsinchu (TW); LIN, Ching-Sung, Hsinchu (TW); HAN, Yung-Lung, Hsinchu (TW); HUANG, Chi-Feng, Hsinchu (TW)
(74) Representative: Uexküll & Stolberg

(56) References cited:
- EP-A1- 2 733 484
- EP-A1- 2 998 582
- WO-A1-2008/024138
- WO-A1-2017/072489
- US-A1- 2014 377 099

## Description

### FIELD OF THE INVENTION

The present disclosure relates to an actuating and sensing module, and more particularly to an actuating and sensing module for use in an electronic device to monitor the environment.

### BACKGROUND OF THE INVENTION

Nowadays, people pay much attention to the devices and methods of monitoring the air quality in the environment. For example, it is important to monitor carbon monoxide, carbon dioxide, volatile organic compounds (VOC), PM2.5, and so on. The exposure of these gases in the environment will cause human health problems or even harm the life. Therefore, it is important for every country to develop and implement the environmental monitoring technology.

As known, portable electronic devices are widely used and applied in the modern lives. In addition, the portable electronic devices are indispensable electronic devices. Accordingly, it is feasible to use the portable electronic device to monitor the ambient air. If the portable electronic device is capable of immediately providing people with the monitored information in the environment for caution, it may help people escape or prevent from the injuries and influence on human health caused by the gas exposure in the environment. In other words, the portable electronic device is suitably used for monitoring the ambient air in the environment.

Generally, the electronic device is additionally equipped with a sensor to monitor the environment and provide information about the environment to the user of the electronic device. However, the monitoring sensitivity and the precision of the sensor are usually not satisfied. For example, since the airflow is transferred to the sensor through natural convection, the amount of the airflow to be monitored by the sensor is neither stable nor uniform. Under this circumstance, the result of monitoring the environment is not accurate. Moreover, since the airflow is transferred to the sensor through natural convection, the response time of the sensor to monitor the environment is much longer. In other words, the real-time monitoring efficacy is low.

Therefore, there is a need of providing a technology of increasing the monitoring accuracy and reducing response time of the sensor.

EP 2 733 484 A1 describes a portable electronic device, such as a mobile phone or a smartphone, having a housing, a gas sensor arranged inside the housing and adapted to measure a property of at least one analyte, and a duct terminating at an opening in the housing for exposing the chemical sensor to the fluid to be analyzed, and an actuator, such as a pump, a fan or a thermally or ultrasonic based pumping unit, to generate a flow within the duct.

EP 2 998 582 A1 discloses a micro-gas pressure driving device which includes a miniature gas transportation module, a covering plate and a tube plate. The miniature gas transportation module includes a convergence plate, a resonance membrane and a piezoelectric actuator. When the piezoelectric actuator is activated to feed a gas into an input tube of the tube plate, the gas is sequentially transferred through a first input chamber, a second input chamber, an inlet, a convergence channel and a central opening of the convergence plate, a central aperture of the resonance membrane, and transferred downwardly through the piezoelectric actuator and an output chamber, and outputted from an output tube of the tube plate. The first input chamber is arranged between the covering plate and the input tube. The second input chamber is defined between the covering plate and the convergence plate. The output chamber is defined between the tube plate and the piezoelectric actuator.

### SUMMARY OF THE INVENTION

An object of the present disclosure provides an actuating and sensing module. The actuating and sensing module is a modular structure of at least one sensor and at least one actuating device. The actuating device can increase the flowing speed and provide the amount of the fluid with stability and uniformity continuously. Since the sensor is provided with the amount of the fluid with stability and uniformity continuously, the time of the sensor in response to the fluid is largely reduced, thereby monitoring the fluid with precision.

The invention is set out in the appended set of claims.

The above contents of the present disclosure will become more readily apparent to those ordinarily skilled in the art after reviewing the following detailed description and accompanying drawings, in which:

### BRIEF DESCRIPTION OF THE DRAWINGS

FIG. 1A is a schematic perspective view illustrating the structure of an actuating and sensing module according to an embodiment of the present disclosure;
FIG. 1B is a schematic cross-sectional view illustrating the structure of the actuating and sensing module according to the embodiment of the present disclosure;
FIG. 1C is a schematic cross-sectional view illustrating the actions of the fluid actuating device of the actuating and sensing module according to the embodiment of the present disclosure;
FIG. 2A is a variant example of the actuating and sensing module according to the embodiment of the present disclosure, in which the carrier is an application-specific integrated circuit (ASIC);
FIG. 2B is another variant example of the actuating and sensing module according to the embodiment of the present disclosure, in which the carrier is a system on chip (SOC);
FIG. 3A is a schematic exploded view illustrating a fluid actuating device used in the actuating and sensing module of the present disclosure;
FIG. 3B is a schematic exploded view illustrating the fluid actuating device of FIG. 3A and taken along another viewpoint;
FIG. 4 is a schematic cross-sectional view illustrating the piezoelectric actuator of the fluid actuating device as shown in FIGS. 3A and 3B;
FIG. 5 is a schematic cross-sectional view illustrating the fluid actuating device as shown in FIGS. 3A and 3B; and
FIGS. 6A to 6E schematically illustrate the actions of the fluid actuating device of the actuating and sensing module according to the embodiment of the present disclosure.

### DETAILED DESCRIPTION OF THE PREFERRED EMBODIMENT

The present disclosure will now be described more specifically with reference to the following embodiments. It is to be noted that the following descriptions of preferred embodiments of this invention are presented herein for purpose of illustration and description only. It is not intended to be exhaustive or to be limited to the precise form disclosed.

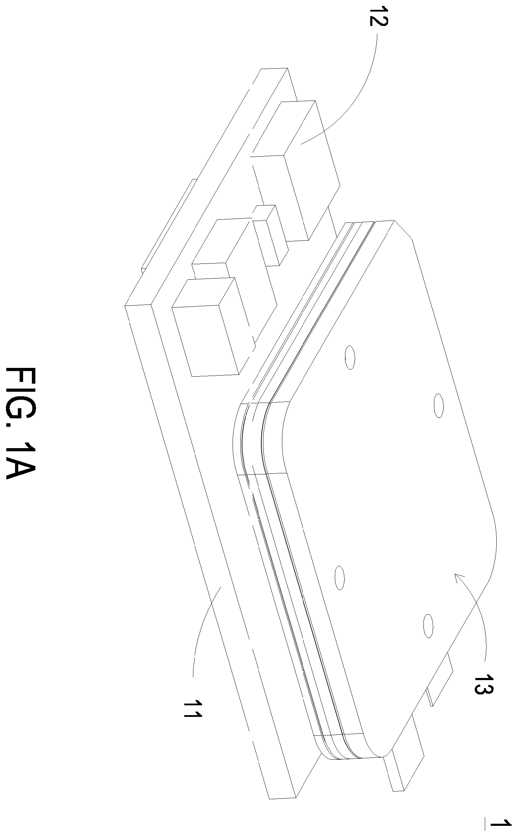

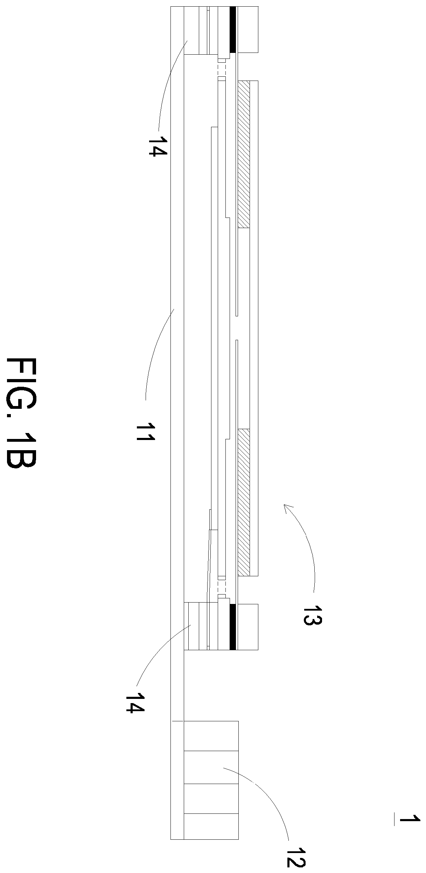

FIG. 1A is a schematic perspective view illustrating the structure of an actuating and sensing module according to an embodiment of the present disclosure. FIG. 1B is a schematic cross-sectional view illustrating the structure of the actuating and sensing module according to the embodiment of the present disclosure. The actuating and sensing module 1 is applied to an electronic device for monitoring associated parameters in the environment. In an embodiment, the actuating and sensing module 1 includes at least one sensor 12 and at least one actuating device 13. The at least one sensor 12 and the at least one actuating device 13 are integrated as the actuating and sensing module 1. The actuating device 13 is disposed on one side of the sensor 12. The actuating device 13 includes at least one guiding channel 14. After the actuating device 13 is enabled, fluid or energy is driven to be transferred through the at least one guiding channel 14. When the fluid or energy is transferred to the sensor 12, the fluid or energy is sensed by the sensor 12. The fluid is a gas or a liquid. The energy is a light energy, an electric energy, a magnetic energy, a sound energy or a chemical energy.

The actuating device 13 is a driving device capable of driving a desired system in response to a control signal. An example of the actuating device 13 includes but is not limited to an electric actuating device, a magnetic actuating device, a thermal actuating device, a piezoelectric actuating device, and a fluid actuating device. For example, the electric actuating device is an electric actuating device of a DC motor, an AC motor or a step motor, the magnetic actuating device is an magnetic actuating device of a magnetic coil motor, the thermal actuating device is a thermal actuating device of a heat pump, the piezoelectric actuating device is a piezoelectric actuating device of a piezoelectric pump, and the fluid actuating device is a fluid actuating device of a gas pump or a liquid pump.

An example of the sensor 12 includes but is not limited to a temperature sensor, a volatile organic compound sensor (e.g., a sensor for measuring formaldehyde or ammonia gas), a particulate sensor (e.g., a PM2.5 particle sensor), a carbon monoxide sensor, a carbon dioxide sensor, an oxygen sensor, an ozone sensor, any other appropriate gas sensor, a humidity sensor, a water content sensor, a substance sensor (e.g., a sensor for measuring compounds or biological substances in liquid or air), a water quality sensor, any other appropriate liquid sensor, a light sensor, or the combination thereof.

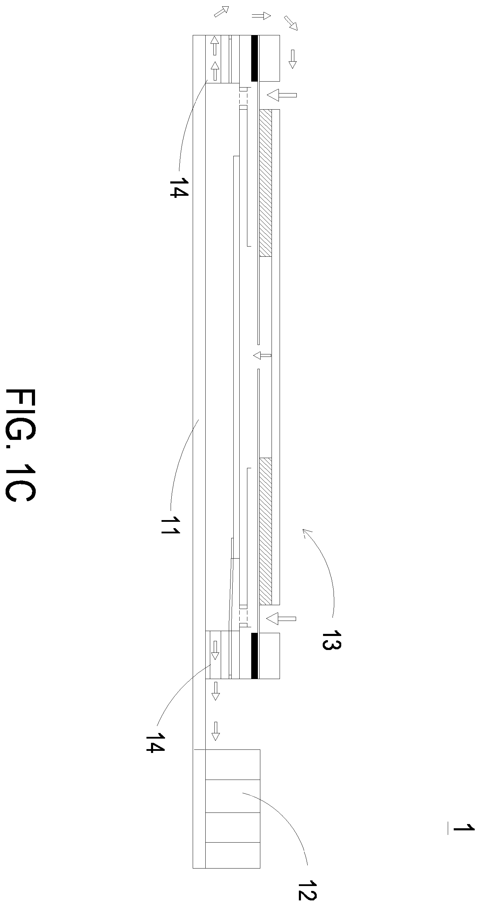

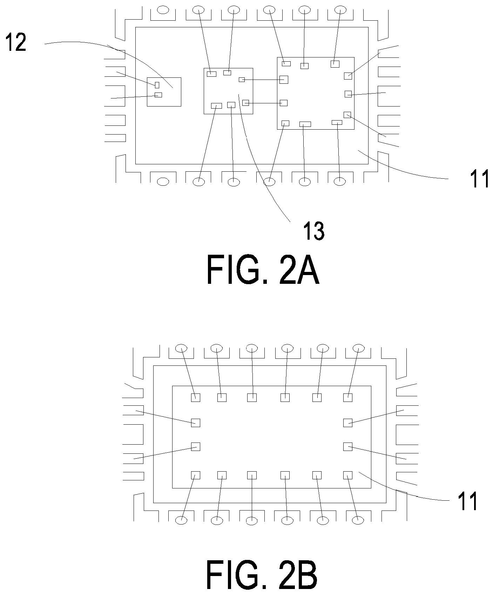

Please refer to FIGS. 1A and 1B. For illustration, the actuating device 13 of the actuating and sensing module 1 is a fluid actuating device. The actions of the fluid actuating device will be described in FIG. 1C. As shown in FIG. 1A, the actuating and sensing module 1 further includes a carrier 11. The carrier 11 is a platform for integrating the sensor 12 with the fluid actuating device 13. For example, the carrier 11 is a substrate such as a printed circuit board (PCB). An array of the sensor 12 and the fluid actuating device 13 is installed on the carrier 11. It is noted that numerous modifications and alterations may be made while retaining the teachings of the invention. FIG. 2A is a variant example of the actuating and sensing module according to the embodiment of the present disclosure. In this variant example, the carrier 11 is an application-specific integrated circuit (ASIC). FIG. 2B is another variant example of the actuating and sensing module according to the embodiment of the present disclosure. In this variant example, the carrier 11 is a system on chip (SOC). The sensor 12 is deposited on the carrier 11. The fluid actuating device 13 is packaged on the carrier 11. That is, the carrier 11, the sensor 12 and the fluid actuating device 13 are combined together as an integral structure. The configuration and type of the carrier 11 are not limited to three examples described above. That is, the carrier 11 may be any other platform for supporting the sensor 12 and the fluid actuating device 13.

The fluid actuating device 13 may be a driving structure of a piezoelectric pump or a driving structure of a micro-electro-mechanical system (MEMS) pump.

Hereinafter, the actions of the fluid actuating device 13 of a piezoelectric pump will be described as follows.

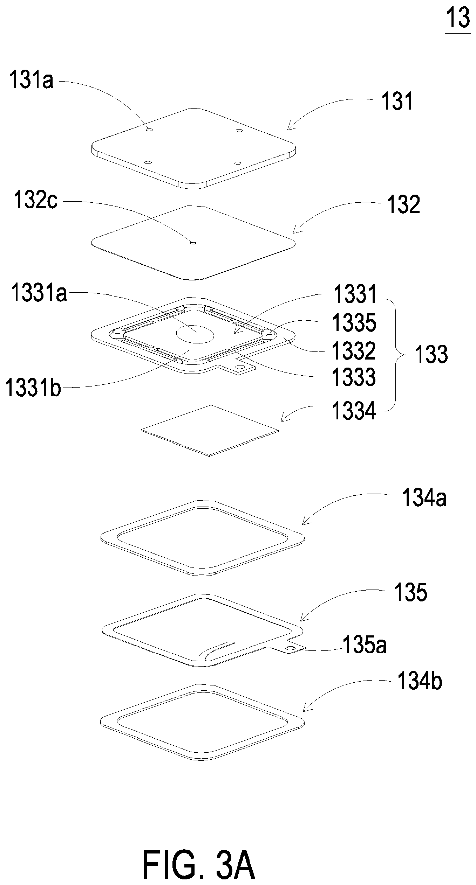

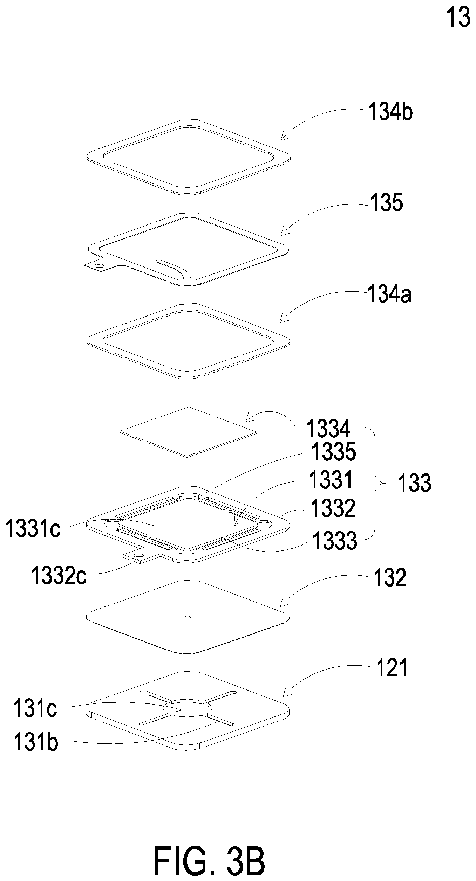

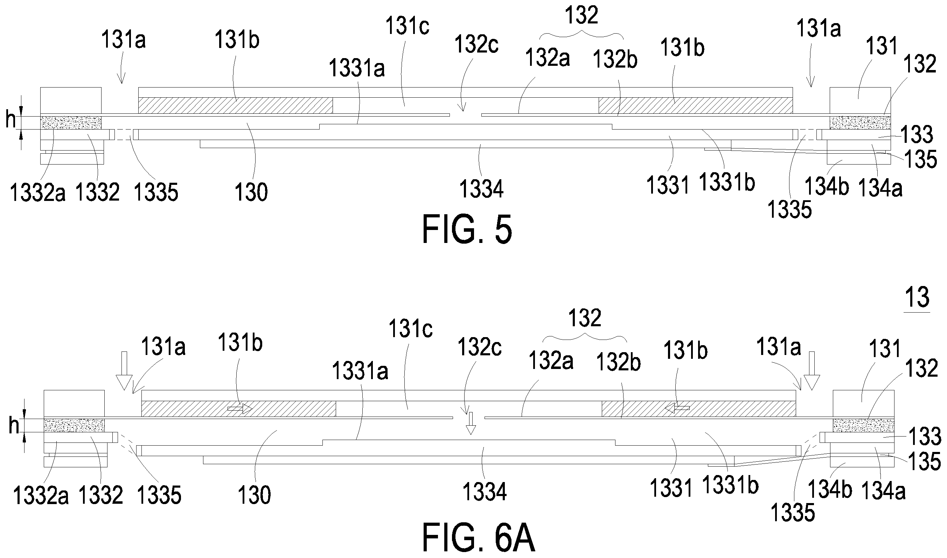

Please refer to FIG. 3A and FIG. 3B. The fluid actuating device 13 includes a fluid inlet plate 131, a resonance plate 132, a piezoelectric actuator 133, a first insulation plate 134a, a conducting plate 135 and a second insulation plate 134b. The piezoelectric actuator 133 is aligned with the resonance plate 132. The fluid inlet plate 131, the resonance plate 132, the piezoelectric actuator 133, the first insulation plate 134a, the conducting plate 135 and the second insulation plate 134b are stacked on each other sequentially. After the above components are combined together, the cross-sectional view of the resulting structure of the fluid actuating device 13 is shown in FIG. 5.

The fluid inlet plate 131 includes at least one inlet 131a. Preferably but not exclusively, the fluid inlet plate 131 includes four inlets 131a. The inlets 131a run through the fluid inlet plate 131. In response to the action of the atmospheric pressure, the fluid can be introduced into the fluid actuating device 13 through the at least one inlet 131a. Moreover, at least one convergence channel 131b is formed on a first surface of the fluid inlet plate 131, and is in communication with the at least one inlet 131a on a second surface of the fluid inlet plate 131. Moreover, a central cavity 131c is located at the intersection of the convergence channels 131b. The central cavity 131c is in communication with the at least one convergence channel 131b, such that the fluid from the at least one inlet 131a would be introduced into the at least one convergence channel 131b and is guided to the central cavity 131c. Consequently, the fluid can be transferred by the fluid actuating device 13. In this embodiment, the at least one inlet 131a, the at least one convergence channel 131b and the central cavity 131c of the fluid inlet plate 131 are integrally formed. The central cavity 131c is a convergence chamber for temporarily storing the fluid. In some embodiments, the fluid inlet plate 131 may be, for example, made of stainless steel. In another embodiment, the depth of the convergence chamber defined by the central cavity 131c is equal to the depth of the at least one convergence channel 131b. The resonance plate 132 may be made of, but not limited to a flexible material. The resonance plate 132 includes a central aperture 132c disposed corresponding to the central cavity 131c of the fluid inlet plate 131. Consequently, the fluid can be transferred through the central aperture 132c. In other embodiments, the resonance plate 132 may be made of, but not limited to a copper material.

The piezoelectric actuator 133 includes a suspension plate 1331, an outer frame 1332, at least one bracket 1333 and a piezoelectric plate 1334. The piezoelectric plate 1334 is attached on a first surface 1331c of the suspension plate 1331. In response to an applied voltage, the piezoelectric plate 1334 would be subjected to a deformation. When the piezoelectric plate 1334 is subjected to the deformation, the suspension plate 1331 is subjected to a bending vibration. The at least one bracket 1333 is connected between the suspension plate 1331 and the outer frame 1332, while the two ends of the bracket 1333 are connected with the outer frame 1332 and the suspension plate 1331 respectively that the bracket 1333 can elastically support the suspension plate 1331. At least one vacant space 1335 is formed between the bracket 1333, the suspension plate 1331 and the outer frame 1332. The at least one vacant space 1335 is in communication with the fluid guiding channel for allowing the fluid to go through. The type of the suspension plate 1331 and the outer frame 1332 and the type and the number of the at least one bracket 1333 may be varied according to the practical requirements. The outer frame 1332 is arranged around the suspension plate 1331. Moreover, a conducting pin 1332c is protruded outwardly from the outer frame 1332 so as to be electrically connected with an external circuit (not shown).

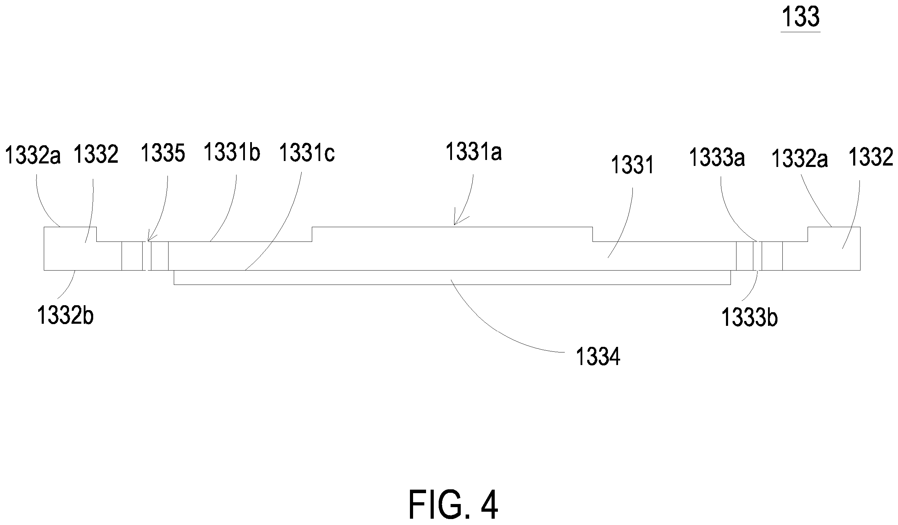

As shown in FIG. 4, the suspension plate 1331 has a bulge 1331a that makes the suspension plate 1331 a stepped structure. The bulge 1331a is formed on a second surface 1331b of the suspension plate 1331. The bulge 1331a may be a circular convex structure. A top surface of the bulge 1331a of the suspension plate 1331 is coplanar with a second surface 1332a of the outer frame 1332, while the second surface 1331b of the suspension plate 1331 is coplanar with a second surface 1333a of the bracket 1333. Moreover, there is a specific depth from the bulge 1331a of the suspension plate 1331 (or the second surface 1332a of the outer frame 1332) to the second surface 1331b of the suspension plate 1331 (or the second surface 1333a of the bracket 1333). A first surface 1331c of the suspension plate 1331, a first surface 1332b of the outer frame 1332 and a first surface 1333b of the bracket 1333 are coplanar with each other. The piezoelectric plate 1334 is attached on the first surface 1331c of the suspension plate 1331. In some other embodiments, the suspension plate 1331 may be a square plate structure with two flat surfaces, but the type of the suspension plate 1331 may be varied according to the practical requirements. In this embodiment, the suspension plate 1331, the at least bracket 1333 and the outer frame 1332 may be integrally formed from a metal plate (e.g., a stainless steel plate). In an embodiment, the length of a side of the piezoelectric plate 1334 is smaller than the length of a side of the suspension plate 1331. In another embodiment, the length of a side of the piezoelectric plate 1334 is equal to the length of a side of the suspension plate 1331. Similarly, the piezoelectric plate 1334 is a square plate structure corresponding to the suspension plate 1331 in terms of the design.

In this embodiment, the first insulation plate 134a, the conducting plate 135 and the second insulation plate 134b of the fluid actuating device 13 are stacked on each other sequentially and located under the piezoelectric actuator 133, as shown in FIG. 3A. The profiles of the first insulation plate 134a, the conducting plate 135 and the second insulation plate 134b substantially match the profile of the outer frame 1332 of the piezoelectric actuator 133. In some embodiments, the first insulation plate 134a and the second insulation plate 134b may be made of an insulating material (e.g. a plastic material) for providing insulating efficacy. In other embodiments, the conducting plate 135 may be made of an electrically conductive material (e.g. a metallic material) for providing electrically conducting efficacy. In this embodiment, the conducting plate 135 may have a conducting pin 135a disposed thereon so as to be electrically connected with an external circuit (not shown).

Please refer to FIG. 5. In an embodiment, the fluid inlet plate 131, the resonance plate 132, the piezoelectric actuator 133, the first insulation plate 134a, the conducting plate 135 and the second insulation plate 134b of the fluid actuating device 13 are stacked on each other sequentially. Moreover, there is a gap h between the resonance plate 132 and the outer frame 1332 of the piezoelectric actuator 133. In this embodiment, the gap h, between the resonance plate 132 and the outer frame 1332 of the piezoelectric actuator 133, may be filled with a filler (e.g., a conductive adhesive) so that a depth from the resonance plate 132 to the bulge 1331a of the suspension plate 1331 of the piezoelectric actuator 133 can be maintained. The gap h ensures the proper distance between the resonance plate 132 and the bulge 1331a of the suspension plate 1331 of the piezoelectric actuator 133, so that the fluid can be transferred quickly, the contact interference is reduced and the generated noise is largely reduced. In some embodiments, alternatively, the height of the outer frame 1332 of the piezoelectric actuator 133 is increased, so that a gap is formed between the resonance plate 132 and the piezoelectric actuator 133.

Please refer to FIG. 3A, FIG. 3B and FIG. 5. After the fluid inlet plate 131, the resonance plate 132 and the piezoelectric actuator 133 are combined together, a movable part 132a and a fixed part 132b of the resonance plate 132 are defined. A convergence chamber for converging the fluid is defined by the movable part 132a of the resonance plate 132 and the fluid inlet plate 131 collaboratively. Moreover, a first chamber 130 is formed between the resonance plate 132 and the piezoelectric actuator 133 for temporarily storing the fluid. Through the central aperture 132c of the resonance plate 132, the first chamber 130 is in communication with the central cavity 131c of the fluid inlet plate 131. The peripheral regions of the first chamber 130 are in communication with the guiding channel 14 through the vacant space 1335 between the brackets 1333 of the piezoelectric actuator 133.

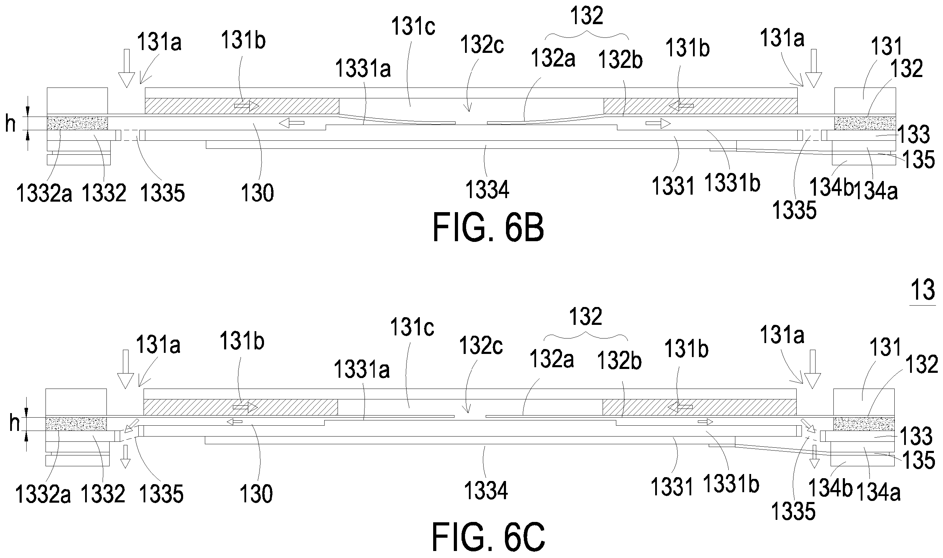

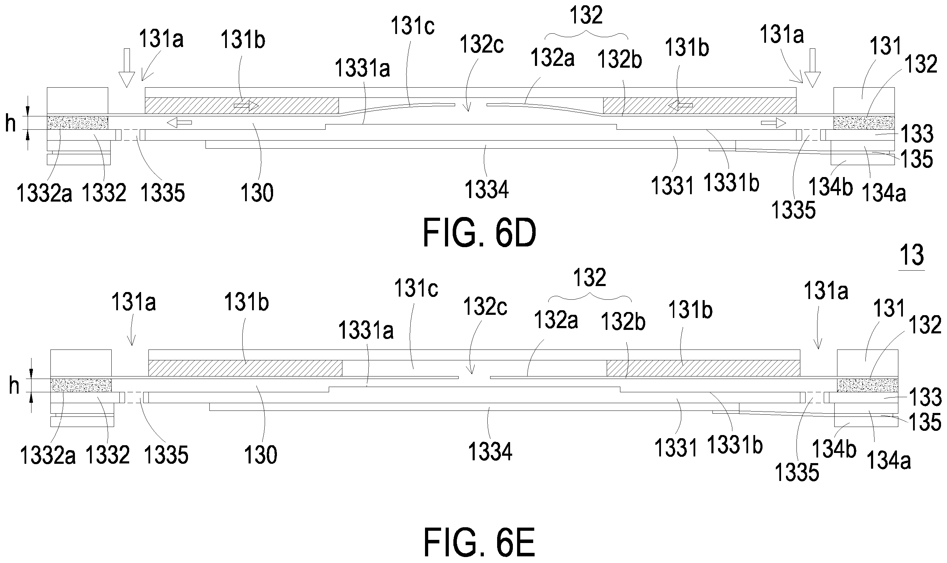

FIGS. 6A to 6E schematically illustrate the actions of the fluid actuating device of the actuating and sensing module according to the embodiment of the present disclosure. Please refer to FIG. 3A, FIG. 3B, FIG. 5 and FIGS. 6A to 6E. The actions of the fluid actuating device will be described as follows. When the fluid actuating device 13 is enabled, the piezoelectric actuator 133 vibrates along a vertical direction in a reciprocating manner by using the bracket 1333 as a fulcrum. Please refer to FIG. 6A. Since the resonance plate 132 is light and thin, the resonance plate 132 vibrates along the vertical direction in the reciprocating manner because of the resonance of the piezoelectric actuator 133 in response to the applied voltage. Besides, a region of the resonance plate 132 spatially corresponding to the central cavity 131c of the fluid inlet plate 131 is also subjected to a bending deformation. The region of the resonance plate 132 corresponding to the central cavity 131c of the fluid inlet plate 131 is the movable part 132a of the resonance plate 132. More specifically, when the piezoelectric actuator 133 vibrates downwardly, the movable part 132a of the resonance plate 132 is subjected to the bending deformation because the movable part 132a of the resonance plate 132 is pushed by the fluid and vibrates in response to the piezoelectric actuator 133. In response to the downward vibration of the piezoelectric actuator 133, the fluid is fed into the at least one inlet 131a of the fluid inlet plate 131. Then, the fluid is transferred to the central cavity 131c of the fluid inlet plate 131 through the at least one convergence channel 131b. Then, the fluid is transferred through the central aperture 131c of the resonance plate 132 spatially corresponding to the central cavity 131c, and introduced downwardly into the first chamber 130. As the piezoelectric actuator 133 is enabled, the resonance of the resonance plate 132 occurs. Consequently, the resonance plate 132 vibrates along the vertical direction in the reciprocating manner. As shown in FIG. 6B, during the vibration of the resonance plate 132 at this stage, the movable part 132a of the resonance plate 132 moves down to contact and attach on the bulge 1331a of the suspension plate 1331 of the piezoelectric actuator 133, and a distance from the fixed part 132b of the resonance plate 132 to a region of the suspension plate 1331 outside the bulge 1331a remains the same. Owing to the deformation of the resonance plate 132 described above, the volume of the first chamber 130 is compressed and a middle communication space of the first chamber 130 is closed. The pressure gradient occurs to push the fluid in the first chamber 130 moving toward peripheral regions of the first chamber 130, and flowing downwardly through the vacant space 1335 of the piezoelectric actuator 133. Referring to FIG. 6C, the movable part 132a of the resonance plate 132 returns to its original position when the piezoelectric actuator 133 vibrates upwardly. Consequently, the volume of the first chamber 130 is continuously compressed to generate the pressure gradient which makes the fluid in the first chamber 130 continuously pushed toward peripheral regions. Meanwhile, the fluid is continuously fed into the at least one inlet 131a of the fluid inlet plate 131, and transferred to the central cavity 131c. Then, as shown in FIG. 6D, the resonance plate 132 moves upwardly, which is cause by the resonance of the upward motion of the piezoelectric actuator 133. That is, the movable part 132a of the resonance plate 132 is also vibrated upwardly. Consequently, it decreases the current of the fluid from the at least one inlet 131a of the fluid inlet plate 131 into the central cavity 131c. At last, as shown in FIG. 6E, the movable part 132a of the resonance plate 132 has returned to its original position. As the embodiments described above, when the resonance plate 132 vibrates along the vertical direction in the reciprocating manner, the gap h between the resonance plate 132 and the piezoelectric actuator 133 is helpful to increase the maximum displacement along the vertical direction during the vibration. In other words, the configuration of the gap h between the resonance plate 132 and the piezoelectric actuator 133 can increase the amplitude of vibration of the resonance plate 132. Consequently, a pressure gradient is generated in the fluid guiding channels of the fluid actuating device 13 to facilitate the fluid to flow at a high speed. Moreover, since there is an impedance difference between the feeding direction and the exiting direction, the fluid can be transmitted from the inlet side to the outlet side. Moreover, even if the outlet side has a gas pressure, the fluid actuating device 13 still has the capability of pushing the fluid to the guiding channel 14 while achieving the silent efficacy. The steps of FIGS. 6A to 6E may be done repeatedly. Consequently, the ambient fluid is transferred by the fluid actuating device 13 from the outside to the inside.

Please refer to FIG. 1C again. After the fluid inlet plate 131, the resonance plate 132, the piezoelectric actuator 133, the first insulation plate 134a, the conducting plate 135 and the second insulation plate 134b are stacked on each other sequentially, the fluid actuating device 13 is assembled. After the fluid actuating device 13 is installed on the carrier 11, the at least one guiding channel 14 is arranged between the fluid actuating device 13 and the carrier 11. The guiding channel 14 is disposed on one side of the sensor 12. When the fluid actuating device 13 is enabled to compress the fluid, the fluid is transferred through the guiding channel 14 along the direction indicated by the arrow (see FIG. 1C). Consequently, the fluid is sensed by the sensor 12. Since the fluid is guided to the sensor 12 by the fluid actuating device 13 and the sensor 12 is provided with the amount of the fluid with stability and uniformity continuously, the time of the sensor in response to the fluid is largely reduced, thereby monitoring the fluid with precision. In other words, the technology of the present disclosure is industrially valuable.

From the above descriptions, the present disclosure provides an actuating and sensing module. The actuating and sensing module is a modular structure of at least one sensor and at least one actuating device. The actuating device can increase the flowing speed and provide the amount of the fluid with stability and uniformity continuously. Since the sensor is provided with the amount of the fluid with stability and uniformity continuously, the time of the sensor in response to the fluid is largely reduced, thereby monitoring the fluid with precision.

While the invention has been described in terms of what is presently considered to be the most practical and preferred embodiments, it is to be understood that the invention needs not be limited to the disclosed embodiment. On the contrary, it is intended to cover various modifications and similar arrangements included within the scope of the appended claims which are to be accorded with the broadest interpretation so as to encompass all such modifications and similar structures.

## Claims

1. An actuating and sensing module (1), comprising:
at least one sensor (12); and
at least one actuating device (13),
**characterized in that** the actuating and sensing module (1) further comprises:
at least one carrier for integrating the at least one sensor (12) with the at least one actuating device (13);
wherein the at least one actuating device (13) is disposed on one side of the at least one sensor (12) and having a central cavity (131c) and two guiding channels (14) sandwiched between the at least one actuating device (13) and the carrier (11), wherein, when the at least one actuating device (13) is enabled to compress a fluid converged to the central cavity (131c), a compressed fluid is transferred to the at least one sensor (12) through one of the two guiding channels (14), so that the compressed fluid is sensed by the at least one sensor (12),
wherein the actuating device (13) comprises a fluid actuating device (13) comprising:
a fluid inlet plate (131) having at least one inlet (131a), at least one convergence channel (131b) and the central cavity (131c) defining a convergence chamber, wherein the at least one inlet (131a) allows the fluid to flow in, and the at least one convergence channel (131b) is disposed corresponding to the at least one inlet (131a) and guides the fluid from the at least one inlet (131a) toward the convergence chamber defined by the central cavity (131c);
a resonance plate (132) having a central aperture (132c) and a movable part (132a), wherein the central aperture (132c) is aligned with the convergence chamber and the movable part (132a) surrounds the central aperture (132c); and
a piezoelectric actuator (133) aligned with the resonance plate (132),
wherein a gap (h) is formed between the resonance plate (132) and the piezoelectric actuator (133) to define a first chamber (130), so that the fluid from the at least one inlet (131a) of the fluid inlet plate (131) is converged to the central cavity (131c) along the at least one convergence channel (131b) and flows into the first chamber (130) through the central aperture (132c) of the resonance plate (132) when the piezoelectric actuator (133) is enabled, whereby the fluid is further transferred through a resonance between the piezoelectric actuator (133) and the movable part (132a) of the resonance plate (132).

2. The actuating and sensing module (1) according to claim 1, wherein the fluid is a gas or a liquid.

3. The actuating and sensing module (1) according to claim 1, wherein the actuating device (13) comprises a piezoelectric actuating device.

4. The actuating and sensing module (1) according to claim 1, wherein the carrier (11) is a substrate, and an array of the sensor (12) and the fluid actuating device (13) is installed on the carrier.

5. The actuating and sensing module (1) according to claim 1, wherein the carrier (11) is an application-specific integrated circuit or a system on chip, and the sensor (12) and the fluid actuating device (13) are packaged on the carrier (11).

6. The actuating and sensing module (1) according to claim 1, wherein the sensor (12) comprises a gas sensor or a liquid sensor.

7. The actuating and sensing module (1) according to claim 1, wherein the sensor (12) comprises at least one selected from the group consisting of an oxygen sensor, a carbon monoxide sensor and a carbon dioxide sensor.

8. The actuating and sensing module (1) according to claim 1, wherein the sensor (12) comprises at least one selected from the group consisting of a temperature sensor, a liquid sensor and a humidity sensor.

9. The actuating and sensing module (1) according to claim 1, wherein the sensor (12) comprises at least one selected from the group consisting of an ozone sensor and a light sensor.

10. The actuating and sensing module (1) according to claim 1, wherein the sensor (12) comprises a particulate sensor or a volatile organic compound sensor.

11. The actuating and sensing module (1) according to claim 1, wherein the piezoelectric actuator (133) comprises:
a suspension plate (1331) having a first surface (1331c) and an opposing second surface (1331b), wherein the suspension plate (1331) is permitted to undergo a bending vibration;
an outer frame (1332) arranged around the suspension plate (1331);
at least one bracket (1333) connected between the suspension plate (1331) and the outer frame (1332) for elastically supporting the suspension plate (1331); and
a piezoelectric plate (1334), wherein a length of a side of the piezoelectric plate (1334) is smaller than or equal to a length of a side of the suspension plate (1331), and the piezoelectric plate (1334) is attached on the first surface (1331c) of the suspension plate (1331), wherein when a voltage is applied to the piezoelectric plate (1334), the suspension plate (1331) is driven to undergo the bending vibration.

12. The actuating and sensing module (1) according to claim 11, wherein the suspension plate (1331) is a square suspension plate with a bulge (1331a), wherein the fluid actuating device (13) further comprises a conducting plate (135), a first insulation plate (134a) and a second insulation plate (134b), wherein the fluid inlet plate (131), the resonance plate (132), the piezoelectric actuator (133), the first insulation plate (134a), the conducting plate (135) and the second insulation plate (134b) are stacked on each other sequentially.

## Patentansprüche

1. Betätigungs- und Messmodul (1), umfassend:
mindestens einen Sensor (12); und
mindestens eine Betätigungsvorrichtung (13),
**dadurch gekennzeichnet, dass** das Betätigungs- und Messmodul (1) ferner umfasst:
mindestens einen Träger zum Integrieren des mindestens einen Sensors (12) mit der mindestens einen Betätigungsvorrichtung (13);
wobei die mindestens eine Betätigungsvorrichtung (13) an einer Seite des mindestens einen Sensors (12) angeordnet ist und eine zentrale Kavität (131c) sowie zwei Führungskanäle (14) aufweist, die zwischen der mindestens einen Betätigungsvorrichtung (13) und dem Träger (11) eingebettet sind, wobei, wenn die mindestens eine Betätigungsvorrichtung (13) aktiviert wird, um ein in die zentrale Kavität (131c) konvergiertes Fluid zu komprimieren, ein komprimiertes Fluid durch einen der beiden Führungskanäle (14) dem mindestens einen Sensor (12) zugeführt wird, so dass das komprimierte Fluid von dem mindestens einen Sensor (12) gemessen wird,
wobei die Betätigungsvorrichtung (13) eine Fluid-Betätigungsvorrichtung (13) aufweist, umfassend:
eine Fluid-Einlassplatte (131) mit mindestens einem Einlass (131a), mindestens einem Konvergenzkanal (131b) und der zentralen Kavität (131c), die eine Konvergenzkammer bilden, wobei der mindestens eine Einlass (131a) das Einströmen des Fluids ermöglicht, und der mindestens eine Konvergenzkanal (131b) entsprechend dem mindestens einen Einlass (131a) angeordnet ist und das Fluid von dem mindestens einen Einlass (131a) in Richtung der durch die zentrale Kavität (131c) definierten Konvergenzkammer leitet;
eine Resonanzplatte (132) mit einer zentralen Öffnung (132c) und einem beweglichen Teil (132a), wobei die zentrale Öffnung (132c) mit der Konvergenzkammer ausgerichtet ist und der bewegliche Teil (132a) die zentrale Öffnung (132c) umgibt; und
einen piezoelektrischen Aktuator (133), der mit der Resonanzplatte (132) ausgerichtet ist,
wobei ein Spalt (h) zwischen der Resonanzplatte (132) und dem piezoelektrischen Aktuator (133) gebildet ist, um eine erste Kammer (130) zu definieren, so dass das Fluid von dem mindestens einen Einlass (131a) der Fluid-Einlassplatte (131) entlang des mindestens einen Konvergenzkanals (131b) zu der zentralen Kavität (131c) konvergiert und durch die zentrale Öffnung (132c) der Resonanzplatte (132) in die erste Kammer (130) strömt, wenn der piezoelektrische Aktuator (133) aktiviert wird, wodurch das Fluid durch eine Resonanz zwischen dem piezoelektrischen Aktuator (133) und dem beweglichen Teil (132a) der Resonanzplatte (132) weiter übertragen wird.

2. Betätigungs- und Messmodul (1) nach Anspruch 1, wobei das Fluid ein Gas oder eine Flüssigkeit ist.

3. Betätigungs- und Messmodul (1) nach Anspruch 1, wobei die Betätigungsvorrichtung (13) eine piezoelektrische Betätigungsvorrichtung umfasst.

4. Betätigungs- und Messmodul (1) nach Anspruch 1, wobei der Träger (11) ein Substrat ist, und ein Array des Sensors (12) und der Fluid-Betätigungsvorrichtung (13) auf dem Träger installiert ist.

5. Betätigungs- und Messmodul (1) nach Anspruch 1, wobei der Träger (11) eine anwendungsspezifische integrierte Schaltung oder ein System-on-Chip ist, und der Sensor (12) und die Fluid-Betätigungsvorrichtung (13) auf dem Träger (11) gepackt sind.

6. Betätigungs- und Messmodul (1) nach Anspruch 1, wobei der Sensor (12) einen Gassensor oder einen Flüssigkeitssensor umfasst.

7. Betätigungs- und Messmodul (1) nach Anspruch 1, wobei der Sensor (12) mindestens einen Sensor umfasst, der aus der Gruppe ausgewählt ist, die einen Sauerstoffsensor, einen Kohlenmonoxidsensor und einen Kohlendioxidsensor umfasst.

8. Betätigungs- und Messmodul (1) nach Anspruch 1, wobei der Sensor (12) mindestens einen Sensor umfasst, der aus der Gruppe ausgewählt ist, die einen Temperatursensor, einen Flüssigkeitssensor und einen Feuchtigkeitssensor umfasst.

9. Betätigungs- und Messmodul (1) nach Anspruch 1, wobei der Sensor (12) mindestens einen Sensor umfasst, der aus der Gruppe ausgewählt ist, die einen Ozonsensor und einen Lichtsensor umfasst.

10. Betätigungs- und Messmodul (1) nach Anspruch 1, wobei der Sensor (12) einen Partikelsensor oder einen Sensor für flüchtige organische Verbindungen umfasst.

11. Betätigungs- und Messmodul (1) nach Anspruch 1, wobei der piezoelektrische Aktuator (133) umfasst:
eine Aufhängungsplatte (1331) mit einer ersten Fläche (1331c) und einer gegenüberliegenden zweiten Fläche (1331b), wobei die Aufhängungsplatte (1331) eine Biegevibration erfahren kann;
einen äußeren Rahmen (1332), der um die Aufhängungsplatte (1331) herum angeordnet ist;
mindestens eine Halterung (1333), die zwischen der Aufhängungsplatte (1331) und dem äußeren Rahmen (1332) verbunden ist, um die Aufhängungsplatte (1331) elastisch zu lagern; und
eine piezoelektrische Platte (1334), wobei die Länge von einer Seite der piezoelektrischen Platte (1334) kleiner oder gleich einer Länge von einer Seite der Aufhängungsplatte (1331) ist, und die piezoelektrische Platte (1334) an der ersten Fläche (1331c) der Aufhängungsplatte (1331) angebracht ist, wobei, wenn eine Spannung an die piezoelektrische Platte (1334) angelegt wird, die Aufhängungsplatte (1331) angetrieben wird, um die Biegevibration zu erfahren.

12. Betätigungs- und Messmodul (1) nach Anspruch 11, wobei die Aufhängungsplatte (1331) eine quadratische Aufhängungsplatte mit einer Ausbuchtung (1331a) ist, wobei die Fluid-Betätigungsvorrichtung (13) ferner eine konduktive Platte (135), eine erste Isolationsplatte (134a) und eine zweite Isolationsplatte (134b) umfasst, wobei die Fluid-Einlassplatte (131), die Resonanzplatte (132), der piezoelektrische Aktuator (133), die erste Isolationsplatte (134a), die konduktive Platte (135) und die zweite Isolationsplatte (134b) sequentiell übereinandergestapelt sind.

## Revendications

1. Module d'actionnement et de mesure (1) comprenant:
au moins un capteur (12); et
au moins un dispositif d'actionnement (13),
**caractérisé en ce que** le module d'actionnement et de mesure (1) comprend en outre:
au moins un support pour intégrer ledit au moins un capteur (12) audit au moins un dispositif d'actionnement (13);
dans lequel ledit au moins un dispositif d'actionnement (13) est placé sur un côté dudit au moins un capteur (12) et comporte une cavité centrale (131c) et deux canaux de guidage (14) pris entre ledit au moins un dispositif d'actionnement (13) et le support (11), dans lequel, quand ledit au moins un dispositif d'actionnement (13) comprime un fluide qui converge vers la cavité centrale (131c), un fluide comprimé est transféré audit au moins un capteur (12) via l'un des deux canaux de guidage (14), de sorte que le fluide comprimé est détecté par ledit au moins un capteur (12),
dans lequel le dispositif d'actionnement (13) comprend un dispositif d'actionnement de fluide (13) comprenant:
une plaque d'entrée de fluide (131) ayant au moins une entrée (131a), au moins un canal de convergence (131b) et la cavité centrale (131c) définissant une chambre de convergence, dans laquelle ladite au moins une entrée (131a) permet au fluide d'entrer, et ledit au moins un canal de convergence (131b) est disposé en correspondance avec ladite au moins une entrée (131a) et guide le fluide de ladite au moins une entrée (131a) vers la chambre de convergence définie par la cavité centrale (131c);
une plaque de résonnance (132) ayant une ouverture centrale (132c) et une partie mobile (132a), l'ouverture centrale (132c) étant alignée avec la chambre de convergence et la partie mobile (132a) entourant l'ouverture centrale (132c); et
un actionneur piézoélectrique (133) aligné avec la plaque de résonnance (132),
dans lequel un espace (h) est formé entre la plaque de résonnance (132) et l'actionneur piézoélectrique (133) pour définir une première chambre (130), de sorte que le fluide provenant de ladite au moins une entrée (131a) de la plaque d'entrée de fluide (131) converge vers la cavité centrale (131c) dans ledit au moins un canal de convergence (131b) et entre dans la première chambre (130) par l'ouverture centrale (132c) de la plaque de résonnance (132) quand l'actionneur piézoélectrique (133) est activé, moyennant quoi le fluide est transféré davantage grâce à une résonnance entre l'actionneur piézoélectrique (133) et la partie mobile (132a) de la plaque de résonnance (132).

2. Module d'actionnement et de mesure (1) selon la revendication 1, dans lequel le fluide est un gaz ou un liquide.

3. Module d'actionnement et de mesure (1) selon la revendication 1, dans lequel le dispositif d'actionnement (13) comprend un dispositif d'actionnement piézoélectrique.

4. Module d'actionnement et de mesure (1) selon la revendication 1, dans lequel le support (11) est un substrat, et un ensemble du capteur (12) et du dispositif d'actionnement de fluide (13) est installé sur le support.

5. Module d'actionnement et de mesure (1) selon la revendication 1, dans lequel le support (11) est un circuit intégré spécialisé ou un système sur puce, et le capteur (12) et le dispositif d'actionnement de fluide (13) sont emballés sur le support (11).

6. Module d'actionnement et de mesure (1) selon la revendication 1, dans lequel le capteur (12) comprend un capteur de gaz ou un capteur de liquide.

7. Module d'actionnement et de mesure (1) selon la revendication 1, dans lequel le capteur (12) comprend au moins un élément choisi dans le groupe comprenant un capteur d'oxygène, un capteur de monoxyde de carbone et un capteur de dioxyde de carbone.

8. Module d'actionnement et de mesure (1) selon la revendication 1, dans lequel le capteur (12) comprend au moins un élément choisi dans le groupe comprenant un capteur de température, un capteur de liquide et un capteur d'humidité.

9. Module d'actionnement et de mesure (1) selon la revendication 1, dans lequel le capteur (12) comprend au moins un élément choisi dans le groupe comprenant un capteur d'ozone et un capteur de luminosité.

10. Module d'actionnement et de mesure (1) selon la revendication 1, dans lequel le capteur (12) comprend un capteur de particules ou un capteur de composés organiques volatils.

11. Module d'actionnement et de mesure (1) selon la revendication 1, dans lequel l'actionneur piézoélectrique (133) comprend:
une plaque de suspension (1331) ayant une première surface (1331c) et une deuxième surface opposée (1331b), la plaque de suspension (1331) étant apte à subir une vibration de flexion;
un cadre extérieur (1332) disposé autour de la plaque de suspension (1331);
au moins une console (1333) connectée entre la plaque de suspension (1331) et le cadre extérieur (1332) pour supporter de manière élastique la plaque de suspension (1331); et
une plaque piézoélectrique (1334), dans laquelle une longueur d'un côté de la plaque piézoélectrique (1334) est inférieure ou égale à une longueur d'un côté de la plaque de suspension (1331), et la plaque piézoélectrique (1334) est fixée sur la première surface (1331c) de la plaque de suspension (1331), dans laquelle, quand une tension électrique est appliquée à la plaque piézoélectrique (1334), la plaque de suspension (1331) est entraînée pour subir la vibration de flexion.

12. Module d'actionnement et de mesure (1) selon la revendication 11, dans lequel la plaque de suspension (1331) est une plaque de suspension carrée comportant un renflement (1331a), dans lequel le dispositif d'actionnement de fluide (13) comprend en outre une plaque conductrice (135), une première plaque isolante (134a) et une deuxième plaque isolante (134b), dans lequel la plaque d'entrée de fluide (131), la plaque de résonnance (132), l'actionneur piézoélectrique (133), la première plaque isolante (134a), la plaque conductrice (135) et la deuxième plaque isolante (134b) sont empilés les uns sur les autres de façon séquentielle.
